# EUROPEAN PATENT APPLICATION

(11) **EP 2 883 494 A1**
(43) Date of publication of application: **17.06.2015**
(21) Application number: 13196882.8
(22) Date of filing: 12.12.2013
(51) Int. Cl.: A61B 5/04, A61B 5/048, A61B 5/00

(54) **Sound stimulator for memory enhancement**

(71) Applicant: Oticon A/S, 2765 Smørum (DK)
(72) Inventor: Lunner, Thomas, DK-2765 Smørum (DK)
(74) Representative: Nielsen, Hans Jørgen Vind

(57) **Abstract**

The present invention regards an in-ear stimulation device (10, 10'; 10a) comprising at least one in-ear sensor (12, 14), a control unit (16), and an output transducer (18). The in-ear stimulation device (10, 10'; 10a) is configured to be worn at or on an ear (26) of a human user (28) and at least partly in an ear canal (30) of the user (28). The at least one in-ear sensor (12, 14) is arranged and configured to measure electrical activity signals (32) indicative of an electroencephalogram (EEG) of a user (28) wearing the device (10, 10'; 10a). The control unit (16) is configured to receive the electrical activity signals (32) and to detect (33) a presence of a slow oscillation (34), which is in a frequency range between 0.5 Hz and 1 Hz, in the electrical activity signals (32). The control unit (16) is further configured to trigger, in response to detecting (33) the presence of a slow oscillation (34), a generation of at least one stimulus impulse (36). The output transducer (18) is configured to receive the at least one stimulus impulse (36) and to generate a corresponding output sound (38).

## Description

The invention regards an in-ear stimulation device comprising at least one in-ear sensor, a control unit and an output transducer and configured to measure electrical activity signals indicative of an electroencephalogram (EEG) of a user, which are analysed and used to generate an output sound corresponding to slow oscillations detected in the electrical activity signals.

Brain activity oscillations reflect synchronized activity that organizes information processing and communication in neuronal cortical networks in a state-dependent manner, see from H.-V. V. Ngo, T. Martinetz, J. Born, and M. Mölle, "Auditory Closed-Loop Stimulation of the Sleep Slow Oscillation Enhances Memory", Neuron 78, 545-553 (2013) [Ngo et al. (2013)], e.g., G. Buzsáki and A. Draguhn "Neuronal oscillations in cortical networks", Science 304 1926-1929 (2004), and F. Varela, J. P. Lachaux, E. Rodriguez, and J. Martinerie, "The brain web: phase synchronization and large-scale integration." Nat. Rev. Neurosci. 2, 229-239 (2001). The brain activity oscillates at different frequencies. Slow oscillations in brain activity, which occur during so-called slow-wave sleep, are critical for retaining memories. [Ngo et al. (2013)] have found that playing sounds synchronized to the rhythm of the slow brain oscillations of people who are sleeping enhances the slow brain oscillations and boosts the memory of the people. This demonstrates an easy and non-invasive way to influence human brain activity to improve sleep and enhance memory.

Brain rhythms regulate information processing in different states to enable learning and memory formation. The <1 Hz sleep slow oscillations hallmark slow-wave sleep and is critical to memory consolidation. [Ngo et al. (2013)] show in sleeping humans that auditory stimulation in phase with the ongoing rhythmic occurrence of slow oscillation up states profoundly enhances the slow oscillation rhythm, phase coupled spindle activity, and, consequently, the consolidation of declarative memory. Stimulation out of phase with the ongoing slow oscillation rhythm remained ineffective. Close-loop in-phase stimulation provides a straight-forward tool to enhance sleep rhythms and their functional efficacy.

In [Ngo et al. (2013)] a method for enhancing memory during sleep is presented. The method performs two auditory stimuli to ears of a user using headphones after the detection of a slow oscillation in the brain activity pattern of the user. The frequency of applying the auditory stimuli corresponds to the oscillation frequency of the slow oscillations in the brain activity of the user. After the application of two auditory stimuli in phase with the slow oscillations, the method is paused 2.5 seconds before a new detection of a slow oscillation can be performed.

EP 2 571 289 A2 shows a hearing aid system comprising two or more electric terminals. The electric terminals are arranged in the surface of a hearing instrument shell where it contacts the skin inside or outside an ear canal of a user. The electric terminals can be configured for the same purpose, e.g., obtaining an ambulatory electroencephalogram (EEG), or for different purposes, e.g., some electric terminals measuring EEG and some electric terminals measuring body temperature. The hearing aid system is used to estimate a user's cognitive status or cognitive load, e.g., based on the user's working memory capacity. The hearing aid system is configured to activate helping systems, such as directional microphones, noise reduction schemes, or time-frequency masking schemes, to reduce the cognitive load, if a cognitive model predicts that the cognitive limit of the user has been exceeded.

WO 2012/097872 A1 describes a personal wearable EEG monitor, which comprises a sensor part, EEG signal analyzer, EEG stimuli controlling means, EEG response detection means, and a classifier. The sensor part has skin surface electrodes for measuring EEG signals from a person. The EEG signal analyzer is adapted for transferring the EEG signal from the EEG sensor part and is adapted for monitoring the EEG signal. The EEG stimuli controlling means are adapted to provide a stimulus to the person, e.g., an auditory stimulus repeated at least two times, to request the person to perform a stimuli creating act, or to identify a stimuli creating ambient sound. The EEG response detection means are adapted to identify an induced response from the EEG signal caused by the stimuli. The classifier is adapted for deciding - based on said induced response - if the skin surface electrodes receive EEG signals. At least one electrode can be adapted to be arranged in an ear canal of a person. The electrode can be arranged on an ear-piece with a permanent shape fitting the ear canal of the person.

It is an object of the invention to provide an improved in-ear stimulation device.

This object is achieved by an in-ear stimulation device comprising at least one in-ear sensor, a control unit and an output transducer. The in-ear stimulation device is configured to be worn at or on an ear of a human user and at least partly in an ear canal of the user. The at least one in-ear sensor is arranged and configured to measure electrical activity signals indicative of an electroencephalogram (EEG) of a user wearing the device. The control unit is configured to receive the electrical activity signals and to detect a presence of a slow oscillation, which is in a frequency range between 0.5 Hz and 1 Hz, in the electrical activity signals. The control unit is further configured to trigger, in response to detecting the presence of a slow oscillation, a generation of at least one stimulus impulse. The output transducer is configured to receive the at least one stimulus impulse and to generate a corresponding output sound.

In-ear sensors can for example be electrodes, such as skin surface electrodes, skin contact electrodes and are preferably dry electrodes. A slow oscillation here corresponds to the most distinct of the brain activity oscillations that hallmark the electroencephalogram (EEG) during slow-wave sleep (SWS) of a user, see from [Ngo et al. (2013)], e.g., I. Timofeev, "Neuronal plasticity and thalamocortical sleep and waking oscillations.", Prog. Brain Res. 193, 121-144, and M. Steriade, "Grouping of brain rhythms in corticothalamic systems", Neuroscience 137, 1087-1106 (2006). The presence of a slow oscillation is preferably detected during a down state of the slow oscillation, meaning 'when the EEG has a minimum voltage value'. The presence of a slow oscillation can also be detected for any negative voltage value below a certain threshold indicative for the presence of a slow oscillation. The stimulus impulse preferably triggers the generation of the corresponding output sound in a way, that the output sound stimulates the user synchronized with an up state of the slow oscillation, meaning that the output transducer generates the output sound when the EEG has a maximum voltage value. Therefore the generation of the output sound is preferably time delayed to the detection of the presence of a slow oscillation, e.g., by a time delayed generation of the stimulus impulse. The output transducer is preferably a speaker, a vibrator of a bone anchored hearing device, or electrodes of a cochlear implant or any other device that is configured to stimulate hearing of the user wearing and using the in-ear stimulation device. The stimulation of the hearing of the user generates a brain activity, as the brain of a human generally processes stimuli from the outside delivered to one of the body internal sensor units, e.g., nerves, sensors in ears, eyes, skin, tongue, nose, or other senor units generating a stimulus that is delivered to the brain.

One aspect of the invention is that the in-ear stimulation device can be worn by a user during sleep and which comprises all functionalities for measuring the electrical activity and for stimulating the user; therefore no more special equipment is needed, e.g., special equipment for synchronizing the electrical activity, i.e., electroencephalogram (EEG) of the user with the sound stimuli, devices for measuring the electrical activity, or devices for generating the output sound and stimulating the user. Another aspect of the invention is that the invention requires only a low power battery, which decreases safety risks compared to general public high voltage AC (110 V to 220 V) needed for prior art EEG hats. The invention also lowers the power consumption. A further aspect of the invention is that the in-ear sensors, i.e., electrodes, do not move significantly during sleep, as the in-ear sensors are fixed in an ear canal of the user, which increases the efficiency of measuring electrical activity signals. The in-ear sensor of the in-ear stimulation device have a better fit to the ear of the user and are fixed, as the in-ear stimulation devices is partly arranged in the ear canal of the user, e.g., by using an earmould. The in-ear stimulation device has almost no physical interference for the user.

In a preferred embodiment of the in-ear stimulation device the control unit is configured to trigger in response to detecting the presence of a slow oscillation a generation of an auditory stimulus impulse sequence of at least two stimulus impulses. The auditory stimulus impulse sequence can, e.g., comprise two, three, four or more consecutive stimulus impulses during which the control unit is adapted not to detect the presence of a slow oscillation. Additionally the auditory stimulus impulse sequence can comprise a detection pause of, e.g., 2 s to 5 s, preferably 2.5 s, following the at least two stimulus impulses in which the control unit is adapted not to detect the presence of a slow oscillation. The stimulus impulse sequence preferably has a frequency in the frequency range between 0.5 Hz and 1 Hz, meaning that stimulus impulses are generated every 1 s to 2 s. The impulses of the auditory impulse sequence are preferably bursts of pink 1/f noise. The duration of the bursts is preferably below 200 ms, such as 100 ms, more preferably 50 ms. The bursts can have a rising time and a falling time, which preferably are symmetric. The rising time is preferably below 50 ms, such as below 25 ms, below 10 ms, or more preferably below 5 ms. The falling time is preferably below 50 ms, such as below 25 ms, below 10 ms, or more preferably below 5 ms. The sound volume of the output sound triggered by the stimulus impulse is preferably calibrated to a sound pressure level of below 80 dB, such as below 70 dB, or more preferably below 55 dB.

In a preferred embodiment of the in-ear stimulation device the control unit is configured to determine a user specific slow oscillation frequency in the electrical activity signals. Preferably the control unit is configured to trigger a generation of an auditory stimulus impulse sequence with the user specific slow oscillation frequency, i.e., with one or more auditory stimuli, which have a time delay to each other, that corresponds to the user specific oscillation frequency. The user specific oscillation frequency in the electrical activity signals can for example be determined by Fourier analysis or other methods known to the person skilled in the art. Preferably the user specific oscillation frequency in the electrical activity signals is determined by means of determining the corresponding time delay between two subsequent extrema, more preferably minima, in the electrical activity signals, with the additional requirement, that minima have to have a value below a predetermined threshold value and maxima have to have a value above a predetermined threshold value. The extrema can be determined by methods known to the person skilled in the art, e.g., by means of determining the first and second derivative of the function describing the electrical activity signals, by means of determining the difference between neighbouring values of the electrical activity signals combined with a minimum difference threshold and/or a requirement that a certain number of subsequent differences between neighbouring values need to be positive or negative to detect a minimum or maximum value, or other methods. The value of the predetermined threshold preferably is adaptive, meaning that the predetermined threshold adapts its value to the lowest value determined in the electrical activity signals for finding a minimum and to the highest value determined in the electrical activity signals for finding a maximum. Preferably the predetermined threshold value has an initial value, e.g., -80 µV for a minimum and +40 µV for a maximum. It is also possible that a lowest predetermined threshold value and a highest predetermined threshold value are used, meaning two threshold values are used at the same time, one predetermined threshold value for detecting the presence of a slow oscillation using a minimum and one predetermined threshold value for detecting the presence of a slow oscillation using a maximum.

The user specific slow oscillation frequency can be determined in a training phase, in which the control unit is preferably configured to only monitor the electrical activity indicative of an encephalogram of the user without triggering stimulus impulses. Preferably, the user specific slow oscillation frequency is determined as an average oscillation frequency value determined from a large number of measurements of the user specific slow oscillation frequency performed during the use of the in-ear stimulation device by a user. A longer usage time of the in-ear stimulation device increases the determined user specific slow oscillation frequency estimate, which allows to improve the synchronicity between the slow oscillations and stimulus impulses. The user specific slow oscillation frequency f can be converted to a user specific time period T by dividing one by the user specific oscillation frequency f, meaning T=1/f. The user specific time period T can be used as a user specific time delay between stimulus impulses to synchronize the frequency of the stimulus impulse sequence with the user specific slow oscillation frequency. The phase of the stimulus impulses and the electrical activity signals can be synchronized by determining amplitudes of the electrical activity signals and triggering the stimulus impulse sequence when the slow oscillation is detected at an extremum, i.e., time delaying the initial stimulus impulse of the stimulus impulse sequence by half the user specific time delay when a minimum is used for detecting the presence of a slow oscillation or time delaying the initial stimulus impulse of the stimulus impulse sequence by one user specific time delay when a maximum is used for detecting the presence of a slow oscillation.

The in-ear stimulation device preferably comprises a memory configured to store data, e.g., bursts of pink 1/f noise, user specific oscillation frequencies, user specific time delays, predetermined thresholds, or other data used by the in-ear stimulation device or units of the in-ear stimulation device.

In a preferred embodiment the control unit is configured to trigger the generation of the auditory stimulus impulse sequence in response to detecting the presence of a slow oscillation and to generate the auditory stimulus impulse sequence after a time delay. Preferably the time delay to the detection of the presence of a slow oscillation is approximately half the user specific time delay or a time delay corresponding to approximately half the oscillation frequency of the detected presence of a slow oscillation, if a minimum of the electrical activity signals is used to detect the presence of the slow oscillation. Preferably the time delay to the detection of the presence of a slow oscillation is approximately one user specific time delay or a time delay corresponding approximately to the oscillation frequency of the detected presence of a slow oscillation, if a maximum of the electrical activity signals is used to detect the presence of the slow oscillation. Approximately here is to be understood as only a small deviation of less than 5%, preferably less than 3% and most preferably less than 1 % of the time period corresponding to the oscillation frequency.

In one embodiment at least one of the in-ear sensors is or comprises a dry electrode. The in-ear sensors can have different sensor types, e.g., skin surface electrodes, skin contact electrodes, dry electrodes, or other sensors which are configured to measure electrical activity signals indicative of an encephalogram (EEG) of a user. Preferably, all of the in-ear sensors are dry electrodes.

In a preferred embodiment the control unit comprises a band-pass filter. The band-pass filter is preferably configured to filter the electrical activity signals between 0.25 Hz and 4 Hz values, meaning that the band-pass filter suppresses the amplitudes of the electrical activity signals with values above 4 Hz or below 0.25 Hz. The band-pass filter can be a unit of the control unit or the band-pass filter can be an algorithm executed on a processing unit of the control unit or in-ear stimulation device.

The control unit is preferably configured to sample the electrical activity signals, with a sampling rate of between 10 Hz and 300 Hz, such as between 100 Hz and 250 Hz, and most preferably with 200 Hz.

In one embodiment, the control unit is configured to trigger the generation of an auditory stimulus impulse sequence or the auditory stimulus impulse each time an amplitude of the electrical activity signals crosses an adaptive negative threshold toward negative values of larger amount than the adaptive negative threshold value. The adaptive negative threshold value preferably has an initial value between -100 µV and -50 µV, more preferably between -90 µV and -75 µV, most preferably -80 µV. The adaptive negative threshold is preferably adjusted every 1 s to 5 s, more preferably every 2 s to 3 s, most preferably every 2 s, according to the largest negative amplitude value of the electrical activity signals within a preceding time interval of 1 s to 10 s, more preferably 5 s to 7 s, most preferably 5 s. The adjustment of the adaptive negative threshold can be performed with constant preceding time intervals or with adaptive preceding time intervals. The time intervals can depend on the largest negative amplitude value of the electrical activity signals and/or the oscillation frequency of the electrical activity signals.

Alternatively, the control unit can be configured to adjust the adaptive negative threshold continuously to the value of the largest negative amplitude and to trigger the generation of an auditory stimulus impulse sequence or the auditory stimulus impulse each time an amplitude of the electrical activity signals crosses an adaptive negative threshold toward negative values of larger amount than the adaptive negative threshold value, while a previous triggering of the generation of the auditory stimulus impulse sequence is cancelled each time a new triggering occurs. In this way, the control unit is configured to find a minimum of the oscillation frequency to detect the presence of a slow oscillation, which is favourable to generate stimulus impulses and output sound in phase with the amplitudes of the electrical activity signals of the user.

Alternatively, the control unit can also be configured to trigger the generation of an auditory stimulus impulse sequence or the auditory stimulus impulse each time an amplitude of the electrical activity signals crosses an adaptive positive threshold toward positive values of larger amount than the adaptive positive threshold value.

Preferably, at least the part of the in-ear stimulation device which is configured to be worn in an ear canal of the user has a form of an earmould configured to fit the ear canal of the user. Also the whole in-ear stimulation device can be integrated in an earmould. Most preferably at least the in-ear sensor or in-ear sensors are arranged in an ear canal of the user. Preferably, the in-ear sensors are integrated in the earmould in a way that the in-ear sensors can contact the skin in an ear canal of the user. The earmould is preferably produced in a stereolithography (SLA) process. Preferably the SLA produced earmoulds comprise the in-ear sensors, e.g., Ear EEG electrodes or the like.

In a preferred embodiment, the in-ear stimulation device is a hearing aid. The in-ear stimulation device as a hearing aid comprises one or more microphones. The microphone or microphones are configured to receive sound from the environment and to generate electrical sound signals according to the sound received from the environment. The control unit of the in-ear stimulation device is preferably configured to receive the electrical sound signals and process the electrical sound signals by generating an output sound signal. The control unit can also be configured to generate a sound interference signal, which is adapted to destructively interfere with environment sound received by the microphone or microphones. The output transducer of the in-ear stimulation device is preferably configured to generate an output sound corresponding to the output sound signal or sound interference signal generated by the control unit.

A further aspect of the invention is that the sleep of a user can be enhanced by suppressing sound of the environment, e.g., airplane noise, street noise, train noise, ultra high noise of devices in standby-mode, ultra high noise of devices, music noise, or the like. This is especially useful if a user intends to sleep when riding an airplane, a train, a bus, a ship or the like and a monotonous environment sound with noise can be disturbing for a healthy sleep of the user.

The in-ear stimulation device can also comprise an interface configured to connect the in-ear stimulation device to one or more hearing aids, or a communication device, such as a mobile phone, a personal computer, an alarm, or the like. The interface can for example be a wired interface, e.g., a wire, a cable, or the like, or a wireless interface, e.g., a Bluetooth transceiver, a wireless transceiver, or the like. The interface is preferably configured to receive electrical signals, e.g., data signals and/or sound signals, from the devices connected to the interface. The control unit is preferably configured to process the electrical signals received by the interface. Electrical signals received by the interface can for example cause the in-ear stimulation device to stimulate the hearing of the user, e.g., by an alarm sound for waking up the user at a time specified by an alarm, by a sleep supporting tune like water wave sounds, or the like.

In one embodiment the in-ear stimulation device is configured to detect when the user using the in-ear stimulation device during sleep is in a sleep phase favourable to wake up the user, e.g., by detecting an oscillation pattern indicating a favourable wake up phase in the electrical activity signals. The in-ear stimulation device is preferably configured to wake up the user in sleep phases favourable to wake up the user, e.g., by stimulating the hearing of the user with a wake up tune, alarm sound, or the like.

Preferably the in-ear stimulation device is powered by a battery. The battery preferably has a low voltage, meaning a voltage in the range of 1 V to 5 V, such as between 1.2 V and 3 V, most preferably between 1.35 V and 1.65 V.

In a preferred embodiment the in-ear stimulation device is used to stimulate the hearing of a user during sleep. The in-ear stimulation device can also be configured to stimulate the user during sleep, e.g., delivering other stimulation pulses that stimulate the electrical activity signals of the user than auditory stimuli.

The in-ear stimulation device can be operated as a stand alone device or in combination with a second in-ear stimulation device as a binaural in-ear stimulation device. If the in-ear stimulation device is operated binaurally, meaning as a binaural in-ear stimulation device, the in-ear stimulation devices of the binaural in-ear stimulation device are preferably wirelessly connected. The wirelessly connected binaural in-ear stimulation devices are preferably configured to exchange data, e.g., electrical activity signals indicative of electroencephalograms (EEG) of the user, sound signals, data signals, user specific oscillation frequencies, user specific time delays, or the like. Preferably each of the in-ear stimulation devices of the binaural in-ear stimulation device is arranged at or on an ear of the user and at least partly in the respective ear canal of the user, e.g., one in-ear stimulation device is arranged on or at the left ear of the user and the other in-ear stimulation device is arranged on or at the right ear of the user.

In a preferred embodiment the control unit of the in-ear stimulation device comprises a processor unit. The processor unit is preferably configured to be able to execute a phase-locked loop algorithm. The phase-locked loop algorithm preferably is a method to synchronize the phase of the stimulus impulses and the phase of the slow oscillation. The phase-locked loop algorithm is preferably adapted to determine a user specific oscillation frequency and/or a user specific time delay, e.g., as described above. The phase-locked loop algorithm is further preferably adapted to apply the user specific time delay to the generation of the stimulus impulse when the presence of a slow oscillation is detected using a maximum and to apply half a user specific time delay to the generation of the stimulus impulse when the presence of a slow oscillation is detected using a minimum, so that the triggering of the stimulus impulse generates a stimulus impulse in phase with a positive amplitude of the slow oscillation, i.e., a slow oscillation up state. The stimulus impulse preferably causes the output transducer to generate an output sound in phase with the slow oscillation up state in the electrical activity signals.

The invention further resides in a method for auditory stimulation. The method is preferably performed using the in-ear stimulation device. The method comprises the following steps. Generate electrical activity signals indicative of an encephalogram (EEG) of a user. Sample the electrical activity signals with a sample rate of between 10 Hz and 500 Hz, such as between 100 Hz and 300 Hz, preferably with a sample rate of 200 Hz. The sampling can be optional. Detect a presence of a slow oscillation, which is in a frequency range between 0.5 Hz and 1 Hz, in the electrical activity signals. Trigger, in response to detecting the presence of a slow oscillation, a generation of at least one stimulus impulse. Generate an output sound corresponding to the stimulus impulse.

A preferred embodiment of the method comprises a step of determining an oscillation frequency in the electrical activity signals. The method preferably comprises a step of performing a stimulation mode if the oscillation frequency is below 1 Hz and performing a monitoring mode if the oscillation frequency is above 1 Hz. The monitoring mode comprises a step of determining an oscillation frequency in the electrical activity signals until the oscillation frequency is below 1 Hz and activating the stimulation mode when the oscillation frequency is below 1 Hz.

The stimulation mode preferably comprises the following steps. Band-pass filter the electrical activity signals between 0.25 Hz and 4 Hz, suppressing electrical activity signals above 4 Hz and below 0.25 Hz. Trigger a stimulus impulse when an amplitude of the electrical activity signals crosses an adaptive negative threshold toward negative values of larger amount than an adaptive negative threshold value, which also corresponds to the detection of the presence of a slow oscillation. Adjust the adaptive negative threshold value to the value with largest negative amount, if the value of largest negative amount is of larger amount than the adaptive negative threshold value. An initial adaptive negative threshold value can for example be between -100 µV and -50 µV, is preferably between - 90 µV and -75 µV, and is most preferably -80 µV. Determine an oscillation frequency of the slow oscillation by determining the corresponding time delay between two up-states or two down-states of the slow oscillation, meaning the time delay between two maxima or two minima of the slow oscillation. Generate a stimulus impulse with a time delay to the detection of the presence of a slow oscillation that corresponds to half the time delay determined for a slow oscillation. Generate the output sound corresponding to the stimulus impulse. The method can also be adapted to generate an auditory stimulus impulse sequence, e.g., comprising two or more stimulus impulses and a detection pause for the time of the auditory stimulus impulse sequence. An additional detection pause can follow the stimulus impulse and/or stimulus impulse sequence, in which the method is adapted not to detect the presence of a slow oscillation.

In one embodiment at least the part of the in-ear stimulation device arranged in an ear canal of the user comprises a vent or allows a venting pathway. The vent or venting pathway is configured to allow an air flow between a tympanic membrane of an ear canal of the user and the outside of the ear to prevent, e.g., an occlusion effect or the like.

In the present context, a "hearing aid" refers to a device, such as e.g. a hearing instrument, a listening device or an active ear-protection device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears.

A "hearing aid" further refers to a device such as an earphone or a headset adapted to receive audio signals electronically, possibly modifying the audio signals and providing the possibly modified audio signals as audible signals to at least one of the user's ears. Such audible signals may e.g. be provided in the form of acoustic signals radiated into the user's outer ears, acoustic signals transferred as mechanical vibrations to the user's inner ears through the bone structure of the user's head and/or through parts of the middle ear as well as electric signals transferred directly or indirectly to the cochlear nerve and/or to the auditory cortex of the user.

A hearing aid may be configured to be worn in any known way, e.g. as a unit arranged behind the ear with a tube leading air-borne acoustic signals into the ear canal or with a loudspeaker arranged close to or in the ear canal, as a unit entirely or partly arranged in the pinna and/or in the ear canal, as a unit attached to a fixture implanted into the skull bone, as an entirely or partly implanted unit, etc. A hearing aid may comprise a single unit or several units communicating electronically with each other. Likewise, the hearing aid may comprise multiple speakers. A purpose of using several speakers may be to reduce vibrations and feedback or to improve LF and HF performance (e.g. by using specific units for each frequency range).

More generally, a hearing aid comprises an input transducer for receiving an acoustic signal from a user's surroundings and providing a corresponding input audio signal and/or a receiver for electronically receiving an input audio signal, a signal processing circuit for processing the input audio signal and an output means for providing an audible signal to the user in dependence on the processed audio signal. Some hearing aids may comprise multiple input transducers, e.g. for providing direction-dependent audio signal processing. In some hearing aids, the receiver may be a wireless receiver.

In some hearing aids, the receiver may be e.g. an input amplifier for receiving a wired signal. In some hearing aids, an amplifier may constitute the signal processing circuit. In some hearing aids, the output means may comprise an output transducer, such as e.g. a loudspeaker for providing an air-borne acoustic signal or a vibrator for providing a structure-borne or liquid-borne acoustic signal. In some hearing aids, the output means may comprise one or more output electrodes for providing electric signals.

An aid may be a part of a "hearing system" that refers to a system comprising one or two hearing aids, and a "binaural hearing system" refers to a system comprising one or two hearing aids and being adapted to cooperatively provide audible signals to both of the user's ears.

Hearing systems or binaural hearing systems may further comprise "auxiliary devices", which communicate with the hearing aids and affect and/or benefit from the function of the hearing aids. Auxiliary devices may be e.g. remote controls, remote microphones, audio gateway devices, mobile phones, public-address systems, car audio systems or music players. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person.

The present invention will be more fully understood from the following detailed description of embodiments thereof, taken together with the drawings in which:
Fig.1 shows a schematic illustration of a first embodiment of the in-ear stimulation device;
Fig. 2 shows a schematic illustration of a second embodiment of the in-ear stimulation device;
Fig. 3 shows a perspective illustration of two in-ear stimulation devices wirelessly connected with each other in a binaural in-ear stimulation device;
Fig. 4 shows a perspective illustration of the first embodiment of the in-ear stimulation device connected to a behind-the-ear (BTE) hearing aid via a cable;
Fig. 5 shows a schematic illustration of an electrical activity signal indicative of an electroencephalogram (EEG) of a user with the presence of a slow oscillation together with a first embodiment of a method of stimulation;
Fig. 6 shows a schematic illustration of an electrical activity signal indicative of an electroencephalogram (EEG) of a user with the presence of a slow oscillation together with a second embodiment of a method of stimulation;
Fig. 7 shows a schematic illustration of a sleeping user stimulated by auditory impulses and a corresponding electroencephalogram (EEG) of the sleeping user;
Fig. 8 shows a block diagram of a method for auditory stimulation;

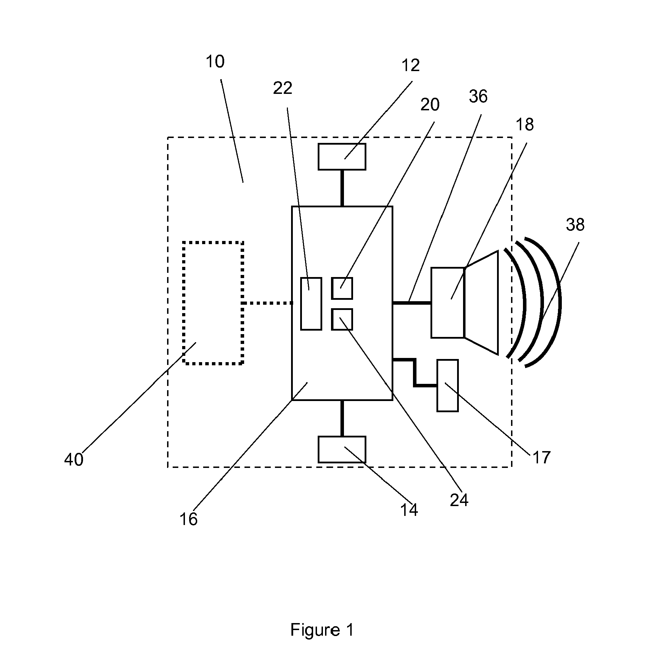

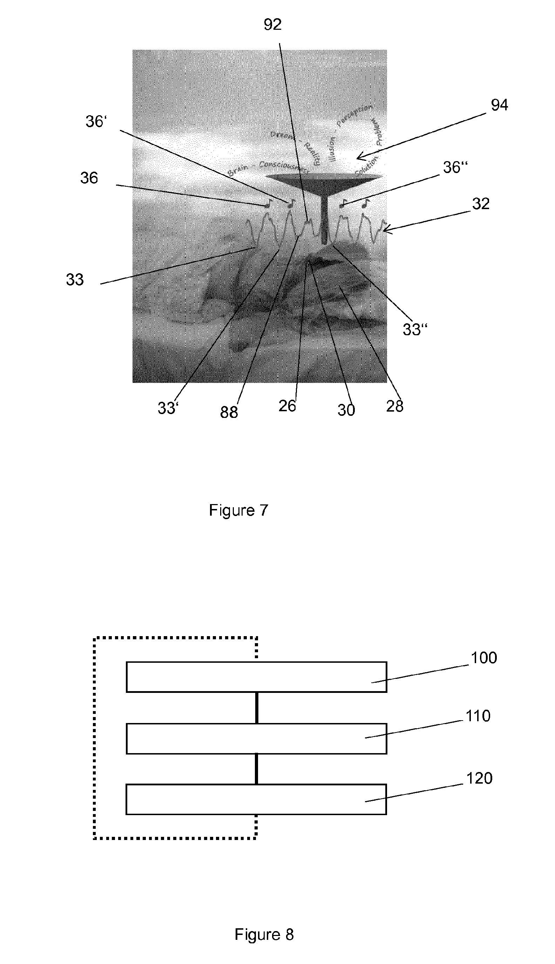

Figure 1 shows a first embodiment of an in-ear stimulation device 10 with a first electrode 12, a second electrode 14, a control unit 16, a battery 17, and a speaker 18. The control unit 16 comprises a memory 20, a processing unit 22, and a band-pass filter 24. The in-ear stimulation device 10 is arranged at an ear 26 of a user 28 (see Fig. 7) with the largest part of the in-ear stimulation device 10 being arranged in an ear canal 30 of the user 28. The electrodes 12 and 14 are in contact with the skin of the ear canal 30.

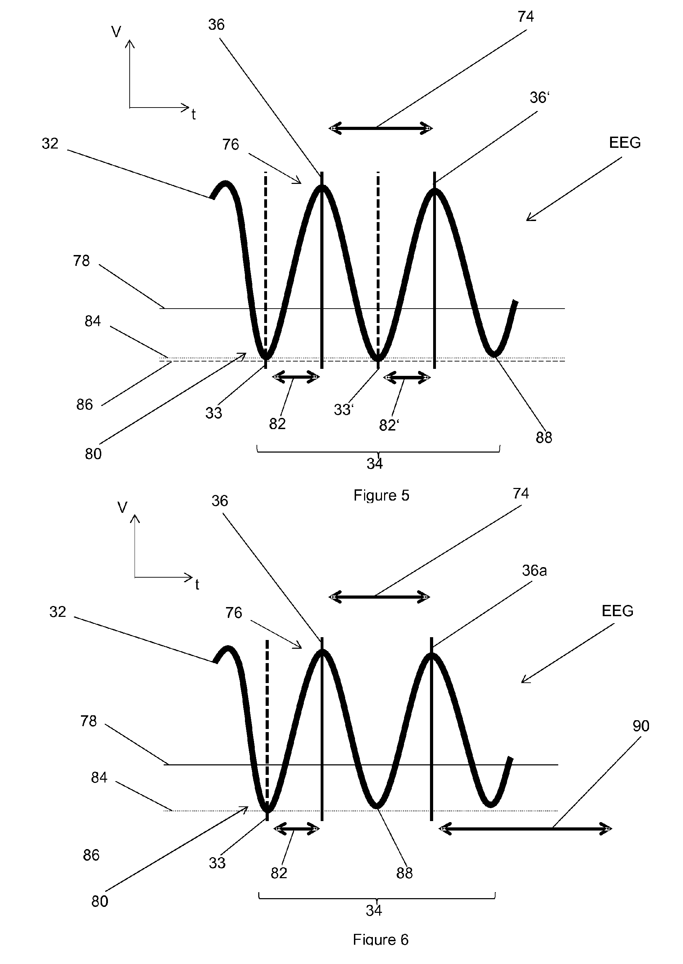

The electrodes 12 and 14 measure electrical activity signals 32 (see Fig. 5 and Fig. 6) indicative of an electroencephalogram EEG of the user 28. The electrical activity signals 32 are received by the control unit 16, which processes the electrical activity signals 32 by detecting 33 the presence of a slow oscillation 34 (see Fig. 5 and Fig. 6). In the embodiment of the in-ear stimulation device 10 presented in Fig. 1 the method presented in Fig. 5 is used to detect the presence of a slow oscillation 34. The control unit 16 triggers, in response to each detection 33, 33' of the presence of a slow oscillation 34, a generation of a stimulus impulse 36, 36'. The speaker 18 receives the stimulus impulse 36, 36' and generates a corresponding output sound 38, which stimulates the hearing of the user 28. The stimulation of the hearing of the user 28 corresponds to a stimulation of the electrical activity of a brain of the user 28. The stimulus impulses 36, 36' are applied in phase with the slow oscillation 34 enhancing the slow oscillation rhythm.

The in-ear stimulation device 10 is powered by the battery 17. The battery 17 has a low voltage between 1.35 V and 1.65 V. The voltage can also be in the range of 1 V to 5 V, such as between 1.2 V and 3 V.

In the following we will give some more details about the functions of the units of the control unit 16.

The band-pass filter 24 filters the electrical activity signals 32 between 0.25 Hz and 4 Hz values, meaning the band-pass filter 24 suppresses the amplitudes of the electrical activity signals 32 with values above 4 Hz or below 0.25 Hz. The band-pass filter 24 can also be an algorithm executed on the processing unit 22.

The processing unit 22 is configured to execute algorithms, to apply outputs of the processing unit 22 and/or the control unit 16 on signals processed by the processing unit 22, and to perform calculations, e.g., for band-pass filtering, for signal amplification, for signal processing, for signal filtering, for determining extrema, like maxima and minima in the electrical activity signals 32, for determining time delays between extrema, for determining oscillation frequencies, or for other functions of the control unit 16. The electrical activity signals 32 are sampled with a sampling rate of 200 Hz. The electrical activity signals 32 can also be sampled with a sampling rate of between 10 Hz and 300 Hz, such as between 100 Hz and 250 Hz.

The memory 20 is used to store data, e.g., bursts of pink 1/f noise, predetermined output sounds, user specific oscillation frequencies, oscillation frequencies in the electrical activity signals 32, user specific time delays, adaptive threshold values, or other data used for the processing of the electrical activity signals 32.

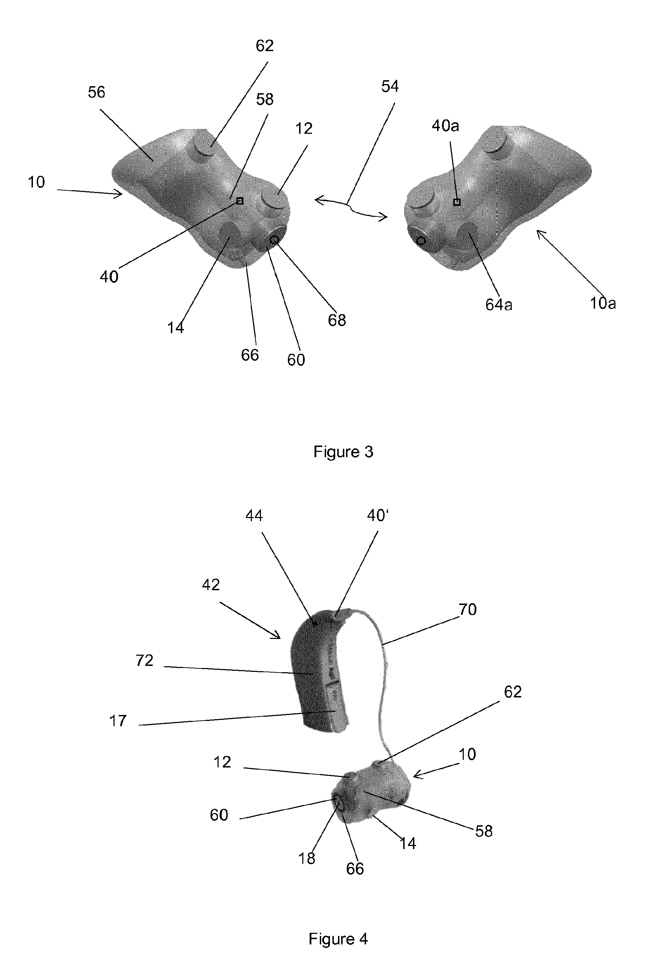

The in-ear stimulation device 10 optionally comprises an interface 40. The interface 40 can be a wired interface 40' (see Fig. 4), e.g., a connector for a wire, a connector for a cable or a connector for a similar line, or a wireless interface 40, e.g., a Bluetooth Transceiver, a wireless transceiver or similar device. The interface 40 is configured to establish a connection to one or more external devices, e.g., a second in-ear stimulation device 10a (see Fig. 3), a hearing aid Behind-The-Ear (BTE) unit 42 (see Fig. 4), a mobile phone, an alarm, a personal computer or other device. The connection between the in-ear stimulation device 10 and the external device connected to the in-ear stimulation device 10 can be used to exchange data, e.g., electrical activity signals 32, sound signals, data signals, bursts of pink 1/f noise, user specific oscillation frequencies, oscillation frequencies in the electrical activity signals 32, user specific time delays, predetermined threshold values, or other data, e.g., used for the processing of the electrical activity signals 32.

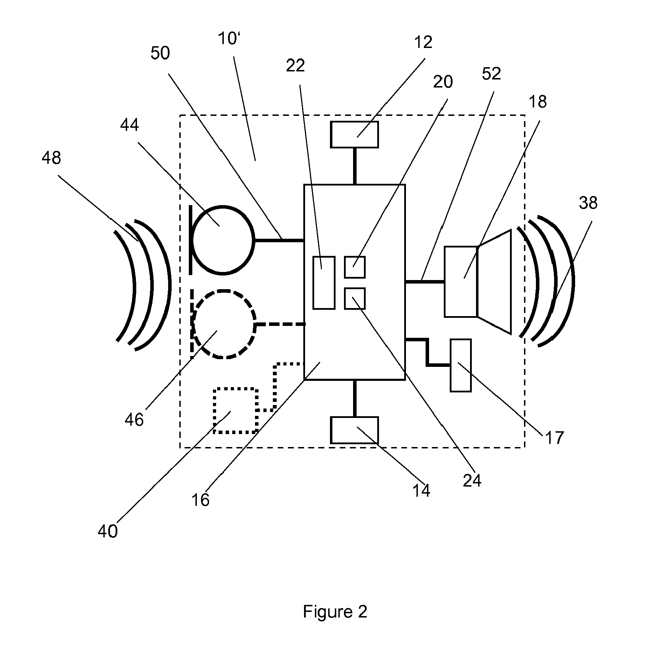

Figure 2 shows a second embodiment of an in-ear stimulation device 10' with a first electrode 12, a second electrode 14, a control unit 16, a speaker 18, a first microphone 44 and a second microphone 46. The control unit 16 comprises a memory 20, a processing unit 22, and a band-pass filter 24. The in-ear stimulation device 10 is arranged at an ear 26 of a user 28 with the largest part being located in an ear canal 30 of the user 28. The electrodes 12 and 14 are in contact with the skin of the ear canal 30. In contrast to the embodiment of the in-ear stimulation device 10 presented in Fig. 1, the in-ear stimulation device 10' presented in Fig. 2 comprises the microphones 44 and 46.

The in-ear stimulation device 10' can be operated in various modes of operation. In a hearing aid mode the in-ear stimulation device 10' is used as a hearing aid. In a sleep enhancement mode the in-ear stimulation device 10' is operated in a way similar to the in-ear stimulation device 10 presented in Fig. 1 with the difference, that sound 48 from the environment received by the microphones 44 and 46 is processed by the in-ear stimulation device 10' to enhance the sleep of the user 28 wearing and using the in-ear stimulation device 10'.

The in-ear stimulation device 10' operating in the hearing aid mode receives sound 48 with the microphones 44 and 46. The microphones 44 and 46 generate electrical sound signals 50, which are provided to the control unit 16. The processing unit 22 of the control unit 16 processes the electrical sound signals 50, e.g., spectral filtering, amplifying, filtering, or other typical processing of sound signals in a hearing aid generating an output sound signal 52. The output sound signal 52 is provided to the speaker 18, which generates an output sound 38 corresponding to the output sound signal 52 which stimulates the hearing of the user 28.

The in-ear stimulation device 10' operating in the sleep enhancement mode receives sound 48 with the microphones 44 and 46. The microphones 44 and 46 generate electrical sound signals 50, which are provided to the control unit 16. The processing unit 22 of the control unit 16 processes the electrical sound signals 50 by generating an output sound signal 52 which is adapted to an interference pattern that allows to generate an output sound 38, which is able to suppress sound 48 from the environment by destructive interference. The output sound signal 52 is provided to the speaker 18, which generates an output sound 38 corresponding to the output sound signal 52, which suppresses the sound 48 reaching the user 28, creating almost complete silence. The electrical sound signals 50 can also be processed in a way to generate an output sound signal 52, which suppresses the sound 48 and at the same time stimulates the hearing of the user 28 with a predetermined sound, e.g., a burst of pink 1/f noise, a sound stored in the memory, such as water wave sounds, whale sounds, or other sounds that enhance the sleeping and/or relaxation experience. The control unit 16 is configured to process the electrical sound signals 50 by generating an output sound signal 52 including a sound 48 suppressing portion and a stimulus impulse 36 portion when the presence of a slow oscillation 34 is detected 33 in the electrical activity signal 32.

The mode of operation of the in-ear stimulation device 10' can be manually selected by the user 28 or automatically selected by the control unit 16, e.g., by detecting a presence of a slow oscillation or other indications that allow to determine that the user 28 is sleeping, or by detecting a sound level above a certain threshold value in a sound from the environment, which in combination with electrical activity signals 32, e.g., high oscillation frequency, received by the electrodes 12 and 14 allows to determine that the user 28 is awake and listening.

Other modes of operation are possible, e.g., a monitoring mode, which is used to determine parameters, e.g., user specific oscillation frequency, user specific time delay, or other parameters.

The in-ear stimulation device 10' is powered by the battery 17. The battery 17 has a low voltage between 1.35 V and 1.65 V. The voltage can also be in the range of 1 V to 5 V, such as between 1.2 V and 3 V.

The in-ear stimulation device 10' can optionally comprise an interface 40. The interface 40 can be used to connect the in-ear stimulation device 10' with other devices, e.g., with a second in-ear stimulation device 10' for binaural operation of the in-ear stimulation device 10'. If the in-ear stimulation device 10' is operated binaurally the two in-ear stimulation devices 10' are connected with each other and the interfaces 40 are configured to exchange data between the two in-ear stimulation devices 10', e.g., electrical activity signals 32, output sound signals 52, electrical sound signals 50, data signals, bursts of pink 1/f noise, user specific oscillation frequencies, oscillation frequencies in the electrical activity signals 32, user specific time delays, predetermined threshold values, or other data, e.g., used for the processing of the electrical activity signals 32.

Figure 3 shows a left in-ear stimulation device 10 and a right in-ear stimulation device 10a wirelessly connected via a wireless link 54. The in-ear stimulation devices 10 and 10a have the form of a stereolithographically (SLA) produced earmould 56 and form a binaural in-ear stimulation device. The control unit 16, the battery 17 (not shown), and the interface 40 are included in an electric circuitry part 58 enclosed by the earmould 56. In contrast to the embodiment of the in-ear stimulation device 10 of Fig. 1 the in-ear stimulation devices 10 and 10a presented in Fig. 3 comprise a third electrode 60, a fourth electrode 62, a fifth electrode 64, and a vent 66. The electrode 60 is a ring-electrode 60 arranged circumferentially around a speaker hole 68, which comprises the speaker 18. The electrodes 12, 14, 60, 62, and 64 receive electrical activity signals 32 when they are in contact with the skin of an ear canal 30 of the user 28. The vent 66 allows an air flow between the ear canal 30 and the outside of the ear 26 to prevent occlusion effects and/or other audio effects. The binaural in-ear stimulation device operates similar to the in-ear stimulation device 10 presented in Fig. 1.

Figure 4 shows a first embodiment of an in-ear stimulation device 10 as presented in Fig. 1 connected via a cable 70 to the interface 40' of a hearing aid Behind-The-Ear (BTE) unit 42. The hearing aid comprises a battery 17, a microphone 44 and electric circuitry 72. In use the in-ear stimulation device 10 is arranged in an ear canal 30 of a user 28 and the hearing aid Behind-The-Ear (BTE) unit 42 is arranged on the ear 26 of the user 28 (cf. Fig. 7).

The hearing aid Behind-The-Ear (BTE) unit 42 can be operated in a hearing aid mode. In the hearing aid mode the microphone 44 of the hearing aid Behind-The-Ear (BTE) unit 42 receives sound 48 from the environment and generates electrical sound signals 50. The electrical sound signals 50 are processed by the electric circuitry 72 of the hearing aid Behind-The-Ear (BTE) unit 42, which generates an output sound signal 52. The output sound signal 52 is transmitted using the wired interface 40' and the cable 70 to the in-ear stimulation device 10. The in-ear stimulation device 10 either processes the output sound signal 52 received by the hearing aid Behind-The-Ear (BTE) unit 42 using the control unit 16 or directly provides the output sound signal 52 to the speaker 18. The speaker 18 generates an output sound 38 corresponding to the output sound signal 52, which stimulates the hearing of the user 28.

The hearing aid Behind-The-Ear (BTE) unit 42 can also be operated in a sleep enhancement mode. The hearing aid Behind-The-Ear (BTE) unit 42 operating in the sleep enhancement mode is configured to receive sound 48 from the environment via the microphone 44. The microphone 44 generates electrical sound signals 50 corresponding to the sound 48 received from the environment. The electrical sound signals 50 are provided to the electric circuitry of the hearing aid Behind-The-Ear (BTE) unit 42, which processes the electrical sound signals 50 by generating an output sound signal 52 which is adapted to an interference pattern that allows to generate an output sound 38, which is able to suppress sound 48 from the environment by destructive interference. The output sound signal 52 is transmitted using the wired interface 40' and the cable 70 to the in-ear stimulation device 10. The in-ear stimulation device 10 either processes the output sound signal 52 received by the hearing aid Behind-The-Ear (BTE) unit 42 using the control unit 16 or directly provides the output sound signal 52 to the speaker 18. The output sound signal 52 is processed in the control unit 16 of the in-ear stimulation device 10, by including an auditory stimulus impulse 36 in the output sound signal 52, when the presence of a slow oscillation 34 is detected 33. The output sound signal 52 can also be processed by generating an output sound signal 52, which suppresses the sound 48 and at the same time stimulates the hearing of the user 28 with a predetermined sound, e.g., a sound stored in the memory, such as water wave sounds, whale sounds, or other sounds that enhance the sleeping and/or relaxation experience, e.g. a tinnitus masking sequence. The speaker 18 generates an output sound 38 corresponding to the output sound signal 52 which stimulates the hearing of the user 28.

Other modes of operation are possible, e.g., a monitoring mode, which is used to determine parameters, e.g., user specific oscillation frequency, user specific time delay, or other parameters.

The hearing aid can also be part of the in-ear stimulation device 10, e.g., similar to the second embodiment of the in-ear stimulation device 10' as presented in Fig. 2.

Figure 5 shows a schematic illustration of an electrical activity signal 32 indicative of an electroencephalogram EEG of the user 28 with a presence of a slow oscillation 34 together with a first embodiment of a method for auditory stimulation of the user 28. In the following the method for auditory stimulation of the user 28 is described.

The brain activity of the user 28 is received by the electrodes 12, 14, 60, 62, and 64, which generate electrical activity signals 32 indicative of an electroencephalogram EEG of the user 28. In Fig. 5 one electrical activity signal 32 of the electrical activity signals 32 is presented. All of the electrical activity signals 32 can be analysed in parallel or a dominant electrical activity signal 32, meaning the signal with the highest absolute values, can be analysed. The electrical activity in the electrical activity signal 32 shows an oscillatory pattern. A user specific oscillation frequency of a slow oscillation 34 corresponding to a user specific time delay 74 between two maxima in the electrical activity signal 32, i.e., slow oscillation up states 76, is determined in a training phase (not shown) and/or continuously determined for each detection 33 of the presence of a slow oscillation 34 in the electrical activity signal 32. An initial adaptive negative threshold 78 has a value of -80 µV. The initial adaptive negative threshold 78 can also have a value between -100 µV and -50 µV, such as between -90 µV and -75 µV.

The method is adapted to detect 33 a presence of a slow oscillation 34 by determining a local minimum voltage value, i.e., a slow oscillation down state 80, of the electrical activity signal 32 which is below the value of the adaptive negative threshold 78. The detection 33 of the presence of the slow oscillation 34 triggers the generation of a stimulus impulse 36 with a time delay 82 to stimulate the electrical activity in a maximum, i.e., the slow oscillation up state 76, of the electrical activity signal 32. The time delay 82 corresponds to half the user specific oscillation frequency of the slow oscillation 34, i.e., half the user specific time delay 74. The method is adapted to adjust the initial adaptive negative threshold 78 to the value of the minimum, so that a new adaptive negative threshold 84 has a higher negative value than the initial adaptive negative threshold 78. After the time delay 82 an auditory stimulus impulse 36 in form of an output sound 38 consisting of a burst of pink 1/f noise of 50 ms, with a 5 ms rising time and a 5 ms falling time is applied via the speaker 18 in the ear canal 30 of the user 28 to stimulate the hearing and the brain activity of the user 28. The duration of the bursts is 50 ms and can also be below 200 ms, such as 100 ms. The rising time and falling time are symmetric. The rising time can also be below 50 ms, such as 25 ms, or 10 ms. The falling time can also be below 50 ms, such as 25 ms, or 10 ms. The sound volume of the output sound 38 triggered by the stimulus impulse 36 is calibrated to a sound pressure level of 55 dB and can also be calibrated to a sound pressure level below 80 dB, such as 70 dB.

The method is configured to detect 33' another presence of a slow oscillation by determining a local minimum voltage value of the electrical activity signal 32 which is below the value of the adaptive negative threshold 84. The detection 33' of the presence of the slow oscillation 34 triggers the generation of a stimulus impulse 36' with a time delay 82' to stimulate the electrical activity in a maximum of the electrical activity signal 32. The time delay 82' is approximately identical to the time delay 82. Approximately here means that the deviation between the time delays is below 1 %. The difference can result from an updated user specific oscillation frequency or corresponding user specific time delay 74, which is determined continuously during the method by determining the time delay between two extrema, i.e., the time delay between two maxima or two minima of the slow oscillation. The adaptive negative threshold 84 is adjusted to the larger negative value of a new adaptive negative threshold 86. After the time delay 82' a stimulus impulse 36' is applied in form of an output sound 38 consisting of a burst of pink 1/f noise of 50 ms, with a 5 ms rising time and a 5 ms falling via the speaker 18 in the ear canal 30 of the user 28 to stimulate the hearing and the brain activity of the user 28.

The method looks for the presence of a slow oscillation, however, the amplitude 88 of the electrical activity signal 32 does not cross the adaptive negative threshold 86. No presence of a slow oscillation 34 is detected. The adaptive negative threshold can be left unaltered or can be adjusted to another value, e.g., the initial value of the adaptive negative threshold 78, the previous value of the adaptive negative threshold 84, or some average between previous values. In this embodiment of the method the adaptive negative threshold 86 is determined as the largest negative value of the preceding 5 s interval.

The method is adapted to continuously look for the presence of slow oscillations until the method is stopped manually by a user or automatically by the method, e.g., when no presence of a slow oscillation has been detected for a certain period of time, e.g., for 1000 s or for 100 s, or when an oscillation frequency has been detected, which suggests, that the slow wave sleep phase has ended, e.g., an oscillation frequency above 2 Hz, more preferably above 4 Hz, that lasts for a period of time significantly larger than a sleep spindle activity time period of the user 28.

Figure 6 shows a schematic illustration of an electrical activity signal 32 indicative of an electroencephalogram EEG of the user 28 with a presence of a slow oscillation 34 together with a second embodiment of a method for auditory stimulation of the user 28. In the following the second embodiment of the method for auditory stimulation of the user 28 is described.

As in the first embodiment of the method presented in Fig. 1, the brain activity of the user 28 is received by the electrodes 12, 14, 60, 62, and 64 which generate electrical activity signals 32 indicative of an electroencephalogram EEG of the user 28. In Fig. 6 one electrical activity signal 32 of the electrical activity signals 32 is presented. All of the electrical activity signals 32 can be analysed in parallel or a dominant electrical activity signal 32, meaning the signal with the highest absolute values, can be analysed. The electrical activity in the electrical activity signal 32 shows an oscillatory pattern. A user specific oscillation frequency of a slow oscillation 34 corresponding to a user specific time delay 74 between two maxima in the electrical activity signal 32, i.e., slow oscillation up states 76, is determined in a training phase (not shown) and/or continuously determined for each detection 33 of the presence of a slow oscillation 34 in the electrical activity signal 32. An initial adaptive negative threshold 78 has a value of -80 µV. The initial adaptive negative threshold 78 can also have a value between -100 µV and -50 µV, such as between -90 µV and -75 µV.

The method is adapted to detect 33 a presence of a slow oscillation 34 by determining a local minimum voltage value, i.e., a slow oscillation down state 80, of the electrical activity signal 32 which is below the value of the adaptive negative threshold 78. The detection 33 of the presence of the slow oscillation 34 triggers the generation of a stimulus impulse sequence, consisting of the two stimulus impulses 36 and 36a, with a time delay 82 to stimulate the electrical activity in maxima, i.e., the slow oscillation up state 76, of the electrical activity signal 32. The time delay 82 corresponds to half the user specific oscillation frequency of the slow oscillation 34, i.e., half the user specific time delay 74. The method is adapted to adjust the initial adaptive negative threshold 78 to the value of the minimum, so that a new adaptive negative threshold 84 has a higher negative value than the initial adaptive negative threshold 78. After the time delay 82 the first auditory stimulus impulse 36 in form of an output sound 38 consisting of a burst of pink 1/f noise of 50 ms, with a 5 ms rising time and a 5 ms falling time is applied via the speaker 18 in the ear canal 30 of the user 28 to stimulate the hearing and the brain activity of the user 28. After the user specific time delay 74 the second auditory stimulus impulse 36a in form of an output sound 38 consisting of a burst of pink 1/f noise of 50 ms, with a 5 ms rising time and a 5 ms falling time is applied via the speaker 18 in the ear canal 30 of the user 28 to stimulate the hearing and the brain activity of the user 28. The second auditory stimulus impulse 36' is followed by a detection pause 90 of 2.5 s. The detection pause can also be between 2 s and 10 s, such as between 2.5 s and 5 s. After the detection pause 90, the method is reinitialised and uses the adaptive negative threshold 84 to detect the presence of a slow oscillation 34.

The Closed-Loop Auditory Stimulation In-Phase with slow oscillation up states 76 induces trains for slow oscillations 34 and enhances declarative memory. Fig. 6 shows the stimulation procedure. The detection 33 of a slow oscillation 34 negative half-wave peak (vertical dashed line) triggers two auditory stimuli 36, 36a (vertical lines), with the first stimulus impulse 36 occurring during the slow oscillation up state 76 of the detected slow oscillation 34 and the second stimulus impulse 36a after a user specific time delay 74, e.g., 1.075 s, after the first stimulus impulse 36.

Figure 7 shows a sleeping user 28. The user 28 is stimulated with auditory stimulus impulses 36, 36', 36" after detection 33, 33', 33" of the presence of a respective slow oscillation 34 following the method described in Fig. 5. The amplitude 88 has a value smaller than the adaptive negative threshold, which prevents the triggering of an auditory stimulus impulse, leading to no stimulation impulse 92. The stimulation of the user during sleep allows to enhance the processing of data during sleep 94.

Figure 8 shows a method for stimulation of the hearing of a user during sleep comprising the steps:
100 generating electrical activity signals 32 indicative of an encephalogram EEG of a user 28,
110 detecting 33 a presence of a slow oscillation 34, which is in a frequency range between 0.5 Hz and 1 Hz, in the electrical activity signals 32,
120 triggering, in response to detecting 33 the presence of a slow oscillation 34, a generation of at least one stimulus impulse 36.

The stimulus impulse can be transmitted to a speaker 18, which is configured to generate an output sound 38 corresponding to the stimulus impulse 36, e.g., a burst of pink 1/f noise. The duration of the bursts is 50 ms and can also be below 200 ms, such as 100 ms. The rising time and falling time are symmetric. The rising time can also be below 50 ms, such as 25 ms, or 10 ms. The falling time can also be below 50 ms, such as 25 ms, or 10 ms. The sound volume of the output sound 38 triggered by the stimulus impulse 36 is calibrated to a sound pressure level of 55 dB and can also be calibrated to a sound pressure level below 80 dB, such as 70 dB.

### Reference signs

- 10: in-ear stimulation device
- 12: first electrode
- 14: second electrode
- 16: control unit
- 17: battery
- 18: speaker
- 20: memory
- 22: processing unit
- 24: band-pass filter
- 26: ear
- 28: user
- 30: ear canal
- 32: electrical activity signals
- EEG: electroencephalogram
- 33: detection of the presence of a slow oscillation
- 34: slow oscillation
- 36: stimulus impulse
- 38: output sound
- 40: interface
- 42: hearing aid Behind-The-Ear (BTE) unit
- 44: first microphone
- 46: second microphone
- 48: sound from the environment
- 50: electrical sound signal
- 52: output sound signal
- 54: wireless link
- 56: earmould
- 58: electric circuitry part
- 60: third electrode
- 62: fourth electrode
- 64: fifth electrode
- 66: vent
- 68: speaker hole
- 70: cable
- 72: electric circuitry
- 74: user specific time delay
- 76: slow oscillation up state
- 78: initial adaptive negative threshold
- 80: slow oscillation down state
- 82: time delay
- 84: adaptive negative threshold
- 86: second adaptive negative threshold
- 88: amplitude with smaller negative value than adaptive threshold
- 90: detection pause
- 92: no stimulation
- 94: processing of data during sleep

## Claims

1. An in-ear stimulation device (10, 10'; 10a) configured to be worn at or on an ear (26) of a human user (28) and at least partly in an ear canal (30) of the user (28), comprising
- at least one in-ear sensor (12, 14) arranged and configured to measure electrical activity signals (32) indicative of an electroencephalogram (EEG) of a user (28) wearing the device (10, 10'; 10a),
- a control unit (16), which is configured
-- to receive the electrical activity signals (32) and to detect (33) a presence of a slow oscillation (34), which is in a frequency range between 0.5 Hz and 1 Hz, in the electrical activity signals (32);
-- to trigger, in response to detecting (33) the presence of a slow oscillation (34), a generation of at least one stimulus impulse (36),
and
- an output transducer (18), which is configured to receive the at least one stimulus impulse (36) and to generate a corresponding output sound (38).

2. An in-ear stimulation device (10, 10'; 10a) according to claim 1, wherein the control unit (16) is configured to trigger in response to detecting (33) the presence of a slow oscillation (34) a generation of an auditory stimulus impulse sequence of at least two stimulus impulses (36), the impulse sequence having a frequency in the frequency range between 0.5 Hz and 1 Hz.

3. An in-ear stimulation device (10, 10'; 10a) according to claim 1 or 2, wherein the control unit (16) is configured to determine a user specific slow oscillation frequency in the electrical activity signal (32) and to trigger a generation of an auditory stimulus impulse sequence, the stimulus impulse sequence having the user specific slow oscillation frequency.

4. An in-ear stimulation device (10, 10'; 10a) according to claim 3, wherein the control unit (16) is configured to trigger the generation of the auditory stimulus impulse sequence in response to detecting (33) the presence of a slow oscillation (34) and wherein the control unit (16) is configured to generate the auditory stimulus impulse sequence after a time delay (82, 82').

5. An in-ear stimulation device (10, 10'; 10a) according to at least one of the claims 1 to 4, wherein the in-ear sensor (12, 14) comprises a dry electrode (12, 14).

6. An in-ear stimulation device (10, 10'; 10a) according to at least one of the claims 1 to 5, wherein the control unit (16) comprises a band-pass filter (24), which is configured to filter the electrical activity signal (32) between 0.25 Hz and 4 Hz values.

7. An in-ear stimulation device (10, 10'; 10a) according to at least one of the claims 1 to 6, wherein the control unit (16) is configured to sample the electrical activity signal (32) with a sampling rate of between 10 Hz and 300 Hz, preferably with 200 Hz.

8. An in-ear stimulation device (10, 10'; 10a) according to at least one of the claims 1 to 7, wherein the control unit (16) is configured to trigger the generation of the auditory stimulus impulse sequence each time an amplitude (80) of the electrical activity signal (32) crosses an adaptive negative threshold (78, 84, 86) toward negative values of larger amount than the adaptive negative threshold value (78, 84, 86).

9. An in-ear stimulation device (10, 10'; 10a) according to claim 8, wherein the control unit (16) is configured to use the adaptive negative threshold (78) with an initial value between -100 µV and -50 µV and to adjust the adaptive negative threshold (78, 84, 86) every 1 s to 5 s according to the largest negative amplitude value (80) of the electrical activity signal (32) within a preceding time interval of 1 s to 10 s.

10. An in-ear stimulation device (10, 10'; 10a) according to at least one of the claims 1 to 9, wherein the control unit (16) is configured to generate stimulus impulses (36) consisting of a burst of pink 1/f noise.

11. An in-ear stimulation device (10, 10'; 10a) according to at least one of the claims 1 to 10, wherein at least the part of the in-ear stimulation device (10, 10'; 10a) which is configured to be worn in an ear canal (30) of a user (28) has a form of an earmould (56) configured to fit the ear canal (30) of the user (28).

12. An in-ear stimulation device (10, 10'; 10a) according to at least one of the claims 1 to 11, wherein the in-ear stimulation device (10, 10'; 10a) is a hearing aid (10') comprising at least one microphone (12, 14) configured to receive sound (38) from the environment, to generate electrical sound signals (50) according to the sound (48) received from the environment, wherein the control unit (16) is configured to receive the electrical sound signals (50) and process the electrical sound signals (50) generating an output sound signal (52), and wherein the output transducer (18) is configured to generate an output sound (38) corresponding to the output sound signal (52).

13. An in-ear stimulation device (10') according to claim 12, wherein the control unit (16) is configured to generate an output sound signal (52), which is adapted to destructively interfere with sound (48) from the environment received by the microphone (44, 46).

14. An in-ear stimulation device (10, 10'; 10a) according to at least one of the claims 1 to 13, wherein the in-ear stimulation device (10, 10'; 10a) is powered by a battery (17).

15. Use of an in-ear stimulation device (10, 10'; 10a) according to at least one of the claims 1 to 14 to stimulate the hearing of a user (28) during sleep.
